# EUROPEAN PATENT APPLICATION

(11) **EP 3 848 449 A1**
(43) Date of publication of application: **14.07.2021**
(21) Application number: 19857333.9
(22) Date of filing: 04.09.2019
(51) Int. Cl.: C12M 3/00, B01F 13/02

(54) **ORGANOID CULTURE SYSTEM AND METHOD FOR STERILISING AN ORGANOID CULTURE SYSTEM**

(30) Priority: 04.09.2018 ES 201830866
(71) Applicant: Universidade de Santiago de Compostela, 15782 Santiago de Compostela (ES); Zumalab, S.L.U., 15703 Santiago de Compostela (A Coruña) (ES)
(72) Inventor: COSTOYA PUENTE, José Antonio, 15782 Santiago de Compostela (ES); ARCE VÁZQUEZ, Víctor Manuel, 15782 Santiago de Compostela (ES); GOLÁN CANCELA, Irene, 15782 Santiago de Compostela (ES); PARDO VÁZQUEZ, José Luis, 15782 Santiago de Compostela (ES); ZUMALAVE RIVAS, José Antonio, 15320 As Pontes (A Coruña) (ES)
(74) Representative: Balder IP Law, S.L.
(86) International application number: PCT/ES2019/070589
(87) International publication number: WO 2020/049206

(57) **Abstract**

The invention relates to a modular sterilizable system for organoid culture, which comprises a culture module with one or more sample wells and an stirring module that includes an air compressor with flow control means, a nozzle for each sample well, a pressure sensor and a controller that acts on the flow control means according to the pressure reading. The method for sterilizing the system consists in decoupling the two modules, removing the culture module and sterilizing it. The system can have a module for monitoring the growth of the organoids, as well as a module for controlling the physiochemical parameters of the culture.

## Description

### TECHNICAL FIELD OF THE INVENTION

The present invention describes an organoid culture system. Furthermore, the present invention also describes a method for sterilizing a culture system.

### BACKGROUND OF THE INVENTION

Various organoid culture systems, also known as three-dimensional culture systems, have been described in the state of the art, in contrast to the traditional two-dimensional monolayer culture.

Qian, X., et al. described in a scientific article (Cell, 165, 1238-1254) in 2016, the development of a miniaturized spinning bioreactor to generate brain-specific organoids from human induced pluripotent stem cells (iPSCs). These organoids incorporated key features of human cortical development, including progenitor zone organization, neurogenesis, gene expression and a human-specific outer radial glia cell layer. The bioreactor was called SpinZ and fits into a standard 12-well tissue culture plate, drastically reducing media consumption and incubator space. The bioreactor also had a stackable version driven by one common motor, which allowed for comparisons of a large number of conditions in parallel (for protocol optimisation) and reducing the incubator space required. However, the system is not modular nor is it a fully sterilizable system. In addition, SpinZ does not incorporate sensors that allow important parameters, such as cell growth, pH, CO₂ and O₂ concentration, and temperature, to be measured in real time.

In a scientific article (Stem Cells Translational Medicine (2017), 6, 622-633), Wilkinson, et al. described a scalable method for the generation of self-assembled human lung organoids. The generation of said organoids was achieved by combining collagen-functionalized alginate beads and human fibroblasts in a 4 ml high □ aspect ratio vessel (HARV) rotational bioreactor. Moreover, the authors also disclose that a GoPro camera can be mounted onto the HARV to characterize the kinetics of organoid formation. However, said invention does not allow standard culture material to be used, nor does it allow for multiple simultaneous culture. The system is not modular either, and although it allows organoid growth to be characterized with the camera that it incorporates, it does not allow this data to be automatically stored or other physiochemical parameters of interest, such as pH, CO₂ and O₂ concentration and the temperature, to be monitored and stored.

Patent application WO2015/184273 A1 discloses a bioreactor system for preparing a cardiac organoid. The bioreactor system includes a first vessel having a hollow interior and an open top, in addition to a cannula having a lumen, a porous ring coupled to the cannula, and a balloon catheter. This invention presents a problem of applicability, since the requirement of a balloon catheter limits organoid production to a single cardiac type. Furthermore, it is not a modular bioreactor nor does it allow for simultaneous culture of different tissues and/or different conditions. This bioreactor also does not incorporate the possibility of using standard culture material. Moreover, it does not allow the organoid growth to be monitored in real time or the data related to the culture's physiochemical parameters of interest to be monitored and stored.

Patent application WO2012/104437 A1 discloses a bioreactor for cell culture on a three-dimensional substrate. It is made up of a conical-shaped culture chamber. The bioreactor is used in tissue engineering for producing tissue grafts, in particular a bone or cartilage graft. This bioreactor does not allow for simultaneous multiple culture or the use of standard culture material. Moreover, the bioreactor of said invention does not allow the organoid growth to be monitored in real time or the data related to physiochemical parameters of interest to be monitored and stored.

In view of the aforementioned cited documents, there is evidently a need for an organoid culture system that is modular and sterilizable, and which allows for simultaneous multiple culture and allows cell growth to be monitored in real time, as well as relevant physiochemical parameters, such as pH, CO₂ and O₂ concentration and temperature, to be monitored and stored. In the present invention, an organoid culture system is described that includes the foregoing features, thus significantly improving the existing systems in the state of the art.

### SUMMARY OF THE INVENTION

The present invention describes an organoid culture system that allows for simultaneous multiple culture (high-throughput), the reuse of standard culture material and equipment, and real-time monitoring of organoid growth and relevant physiochemical parameters. Moreover, the system is modular and one module can be sterilized independently of another.

One aspect of the present invention relates to a sterilizable organoid culture system comprising:
a culture module comprising:
   one or more sample wells;
an stirring module comprising stirring means for each sample well;
characterized in that:
   the stirring module comprises:
   an air compressor system comprising flow control means configured to supply a pressurized air flow at a given pressure;
   a nozzle for each sample well, configured to channel the pressurized air flow to each sample well;
   a pressure sensor and a controller, the controller being configured to act on the flow control means by varying supply power of the flow control means based on a pressure reading provided by the pressure sensor.

Therefore, the present invention describes a system that has numerous advantages over other organoid culture bioreactors described in the state of the art. The bioreactor of the present invention allows for multiple simultaneous culture, which allows experiments with different conditions to be conducted in parallel. Moreover, the present invention comprises a modular system, whereby different modules of the system can be coupled and decoupled. Therefore, firstly, it enables the culture module to be sterilized in its entirety, without the need to sterilize the parts of the culture module separately, this being very convenient for keeping the entire culture sterile. In other words, the culture module, which comprises one or more sample wells, can be removed as a block, without the need to disassemble the components thereof.

The present invention also describes a method for sterilizing an organoid culture system comprising:
providing a system like the one defined above in a stand-by configuration such that the stirring module and the control module are decoupled from the culture module;
removing the culture module;
and subjecting the culture module to sterilization.

### BRIEF DESCRIPTION OF THE FIGURES

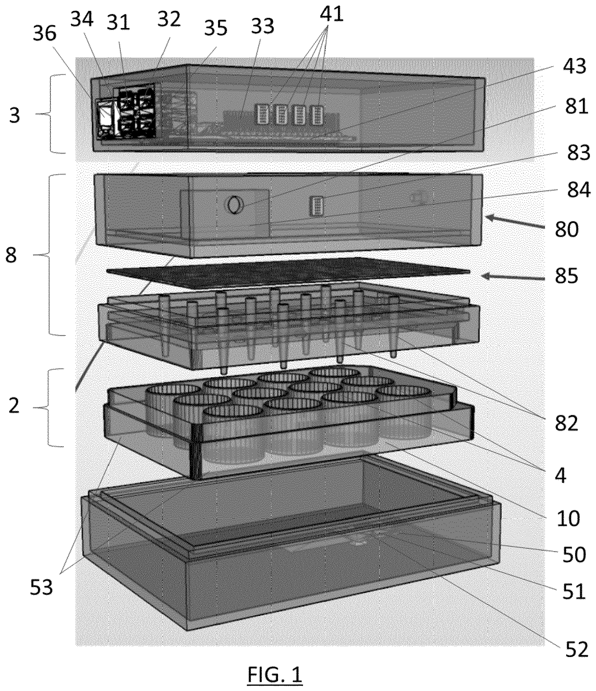

Figure 1 shows an exemplary embodiment of the present invention, wherein the organoid culture system comprises
   a culture module (2) comprising:
   one or more sample wells (4);
   an stirring module (8) comprising stirring means for each sample well (4); characterized in that:
   the stirring module (8) comprises:
   an air compressor system comprising flow control means (81) configured to supply a pressurized air flow at a given pressure;
   a nozzle (82) for each sample well (4), configured to channel the pressurized air flow to each sample well (4);
   a pressure sensor (83) and a controller (84), the controller (84) being configured to act on the flow control means (81) by varying supply power of the flow control means (81) based on a pressure reading provided by the pressure sensor (83).
Figure 2 shows an embodiment of a cover (5) that is placed over the sample wells (4). Figure 2 shows that the cover (5) comprises perforations (51) over each of the sample wells (4) to convey discharge air from each sample well (4) to the outside. The discharge air passes through the perforations (51) to an air chamber (52). Figure 2 also shows the nozzles (82).
Figure 3 shows an embodiment wherein the culture module (2) and the growth monitoring module (50) are comprised in a container (1).
Figure 4 shows an embodiment of the growth monitoring module (50) and the coupling thereof to the sample wells (4) comprised in the culture module (2). In the embodiment illustrated in Fig. 4, the growth monitoring module (50) and culture module (2) are separated by a transparent base (15).
Figure 5 shows a diagram of a motorized system (52) to allow the vision system (511) to be positioned by means of a Cartesian system with two motorized axes, X - Y.

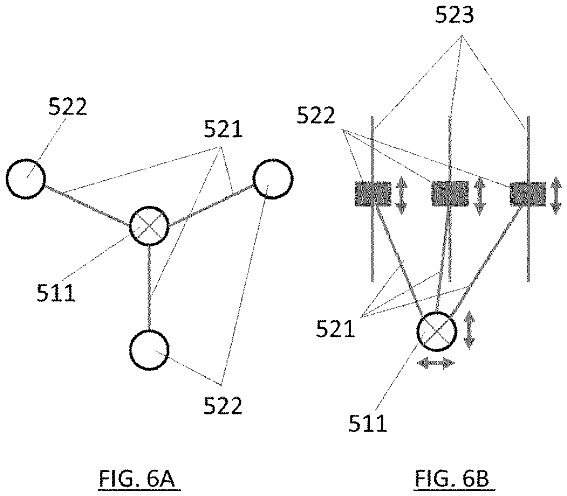

Figures 6A and 6B show a diagram of a motorized system (52) to allow the vision system (511) to be positioned by means of a Delta system with 3 motorized arms.

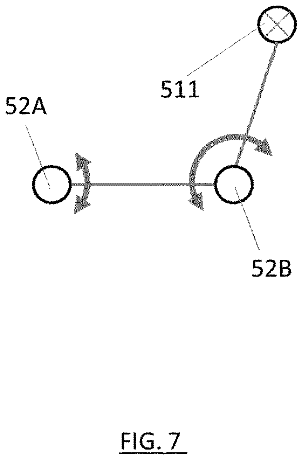

Figure 7 shows a diagram of a motorized system (52) to allow the vision system (511) to be positioned by means of an articulated arm with at least two joints.
Figure 8 shows a possible embodiment for the placement of visual codes (532, 533) that form part of visual guides (53) on a culture plate (104). Figure 8 shows a two-dimensional free identification code, such as QR (532), and two-dimensional markers (533), which allow the corners of the culture plate (104) and the features thereof to be defined.
Figure 9 shows an embodiment of the invention wherein the growth monitoring module (50) comprises diffuse lighting means (54) located at the edges of the filter holder (55).

### DESCRIPTION OF THE INVENTION

The present invention describes an organoid culture system and a method for sterilizing an organoid culture system.

With reference to Fig. 1, in a particular embodiment, the present invention is a sterilizable organoid culture system comprising:
a culture module (2) comprising:
   one or more sample wells (4);
an stirring module (8) comprising stirring means for each sample well (4);
characterized in that:
   the stirring module (8) comprises:
   an air compressor system (80) comprising flow control means (81) configured to supply a pressurized air flow at a given pressure;
   a nozzle (82) for each sample well (4), configured to channel the pressurized air flow to each sample well (4);
   a pressure sensor (83) and a controller (84), the controller (84) being configured to act on the flow control means (81) by varying supply power of the flow control means (81) based on a pressure reading provided by the pressure sensor (83).

The basis of the culture module (2) is the stirring of the culture by air bubbles, which also help to facilitate the gas exchange with the culture medium.

In an embodiment of the invention, the controller (84) is configured so that the pressure reading provided by the pressure sensor (83) corresponds to the pressure transfer/time curve selected for each experiment.

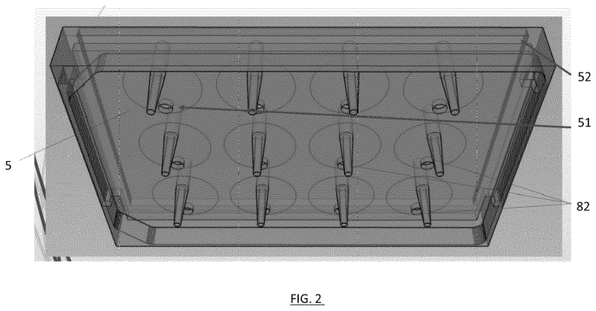

In an embodiment of the invention illustrated in Fig. 2, the stirring module (8) comprises:
a cover (5) comprising discharge means (51, 52) for discharging air from each sample well (4). The outlet of air from the sample wells (4) is carried out through perforations (51) made in the cover (5) over each of the sample wells (4), and it is led to the outside through an air chamber (52) built on the cover (5), to avoid contaminating the culture.

In an embodiment of the invention illustrated in Fig. 1, the stirring module (8) comprises an air filter (85) between each nozzle (82) and the air compressor system (80). According to an embodiment of the invention, the air filter (85) is a HEPA filter.

According to an embodiment of the invention, the air compressor system (80) collects the air from the incubator where the system of the invention is located and drives it towards the nozzles (82), previously passing through the filter (85), that avoids contaminating the culture medium.

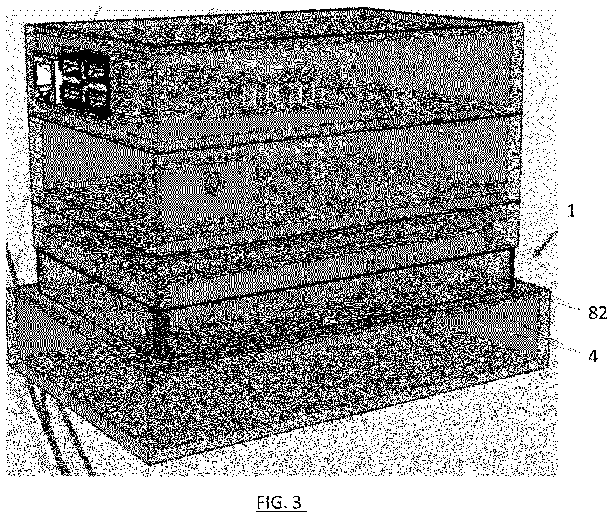

As illustrated in Fig. 3, during the growth of the organoid, the air nozzles (82) remain introduced in the culture medium. The air compressor system (80) generates a constant air flow, which produces bubbling in each of the sample wells (4). The system has a pressure sensor (83) that is used to regulate the speed of the flow control means (81), which can be a compressor, ensuring a constant air flow to the sample wells (4) through the air nozzles (82).

In an embodiment of the invention, the controller (84) is configured to detect a malfunction corresponding to a clogged nozzle (82), a dirty air filter (81), and both. The control system has an air pump control algorithm that, based on the compressor speed data and the pressure read in the chamber prior to the filter (85), allows clogged nozzles (82) or a dirty filter (85) to be detected.

In an embodiment of the invention illustrated in Fig. 1, the sample wells (4) are arranged on support means (10).

The support means (10) allows for the comprehensive manipulation of all the samples wells (4) used for the organoid culture. Preferably, the system described in the present invention can be used in combination with a standard culture plate (104). In a standard culture plate (104), the support means (10) and the sample wells (4) form a single unit.

Thus, in a preferred embodiment, standard culture plates can be used in the organoid culture system described in the present invention, such that the bioreactor is widely applicable and the use thereof will not require special devices for organoid culture, unlike other systems described in the state of the art. Furthermore, the use of standard culture plates allows for simultaneous multiple culture, thus allowing for cell culture under different physiochemical conditions on the same plate, in the same experiment.

In an embodiment of the invention illustrated in Fig. 1, the system comprises:
an environmental sensorization module (43) comprising one or more sensors (41);
wherein said one or more sensors (41) are configured to measure culture-related parameters selected from cell growth, CO₂ concentration, O₂ concentration, pH, temperature, humidity and volatile organic compounds.

According to an embodiment of the invention, the one or more sensors (41) are configured to measure the culture-related parameters in real time.

In addition, the system of the invention allows new sensors to be incorporated to record new information in real time that may be relevant.

In an embodiment of the invention illustrated in Fig. 1, the system comprises:
a growth monitoring module (50) comprising:
a vision system based on individual images of each sample well (4).

The organoid growth is monitored in real time by means of a computer vision system, which obtains individual images of each sample well (4) and processes them to calculate the size thereof.

In an embodiment of the invention illustrated in Fig. 1, the vision system comprises image capturing means (51) selected from means comprising a fixed focus lens, adjustable focus lens, light field technology and multi-camera technology.

With the fixed focus lens, the image capturing means (51) are adjusted to the resting plane of the organoids, in order to perform a growth estimation based on a 2D projection of the culture.

With the adjustable focus lens, the image capturing means (51) allow several images of each sample well to be captured, corresponding to different planes of the culture, improving the growth estimation, since it allows for a 3D reconstruction of the organoid.

With light field technology, the image capturing means (51) allow the growth to be seen in different planes without an adjustable lens, since light field technology allows the focal plane of the capture to be modified without the need for adjustable focus lenses.

With multi-camera technology, the image capturing means (51) comprise several cameras that focus on the culture from different angles, allowing for the 3D reconstruction of the same.

In an embodiment of the invention illustrated in Fig. 1, the growth monitoring module (50) comprises a motorized system (52) for positioning the vision system in each of the sample wells (4). The motorized system (52) is configured to allow the vision system (511) to be positioned three-dimensionally, in other words, at a point in space determined by three coordinates.

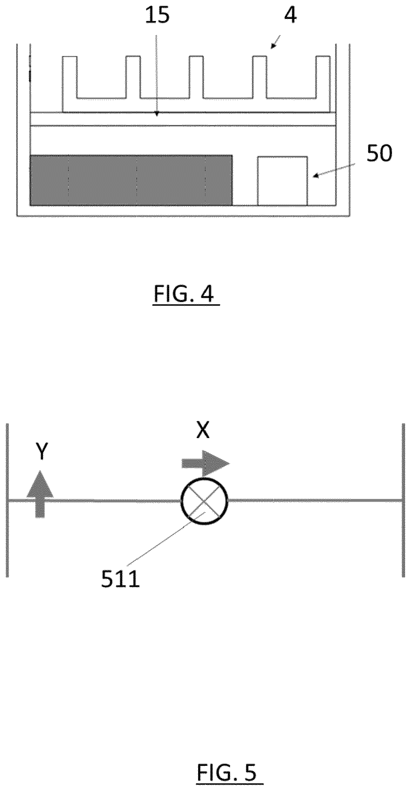

According to different embodiments of the invention, the motorized system (52) comprises a system selected from:
a Cartesian system with two motorized axes, X - Y, as illustrated in Fig. 5, wherein:
   the vision system (511) is installed on a sliding axis (X), which can be driven by means of a gear and motor mechanism;
   the sliding axis (X) is in turn installed on a perpendicular moving carriage (Y), driven by a mechanism, which can be gears and motor, independent of the X axis;
a Delta system with 3 motorized arms wherein:
   the vision system (511) is installed on a support that is connected by three telescopic segments (521) to three carriages (522) configured to slide vertically on respective vertical axes (523), the vertical axes (523) being arranged on the vertices of an equilateral triangle, seen on a horizontal plane;
   the vision system is moved horizontally and vertically by a coordinated movement of the 3 carriages (522) along the vertical axes (523);
an articulated arm with at least two joints (52A, 52B) wherein the vision system (511) is located at a free end of the arm that has at least two rotating joints (52A, 52B), the coordinated movement of which allow the vision system to be positioned. In an embodiment, the articulated arm has a first joint (52A) configured for positioning in a horizontal plane, and a second joint (52B) configured for positioning in a vertical plane.

In addition, in accordance with an embodiment of the invention illustrated in Fig. 1, the motorized system (52) comprises calibrating means for calibrating the positioning of the vision system by means of visual guides (53) attached to a culture plate (104) selected from geometric shapes and visual codes. In an embodiment of the invention, the visual guides (53) are located at the corners of the culture plate (104), to allow a camera of the vision system to be automatically aligned, adapting to variations in location. Visual codes can be one-dimensional or two-dimensional.

According to an embodiment of the invention, the visual guides (53) are encoded and selected from two-dimensional codes and two-dimensional markers (fiducial markers).

The two-dimensional codes, such as QR, allow any type of alphanumeric information to be stored, encoded as points forming a two-dimensional matrix.

The two-dimensional markers (fiducial markers) are also made up of a two-dimensional matrix of points, but unlike two-dimensional codes, the information stored is predefined and usually corresponds to a sequence number for each unique code in a predetermined dictionary. For example, in the case of AprilTag codes, the 36H11 family allows for 518 different codes.

The two-dimensional markers are often used as indices for automatic positioning or detection of predefined elements.

In an embodiment of the invention, a combination of both types of codes is used:
Two-dimensional codes, such as QR, for uniquely identifying the culture medium assigned to a specific experiment;
Two-dimensional markers, for calibrating and adjusting the exact position of the culture medium on the vision system, as well as for encoding other specific data of the culture plate (104) (format, number of sample wells, etc.).

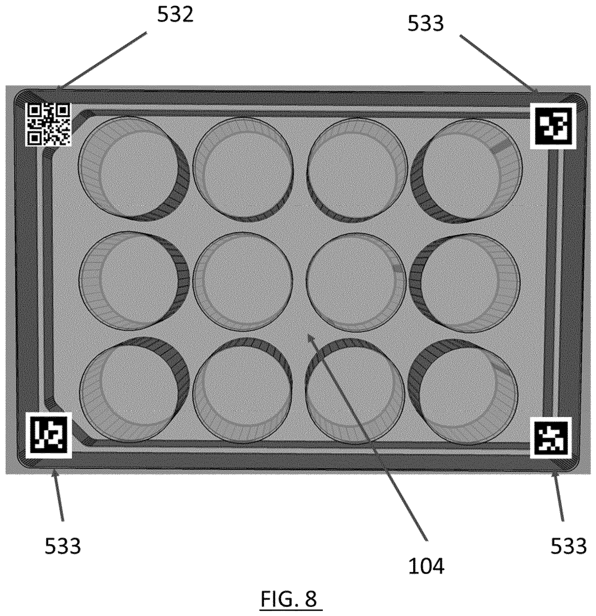

Figure 8 shows a possible embodiment for the placement of these codes on a culture plate (104). The different codes are placed on the corners of the culture plate (104), so that they do not obstruct the view of the cuvettes so as not to interfere with the visual identification of the growth of the culture. On the one hand, a two-dimensional free identification code, such as QR (532), is used to encode the culture support code and be able to associate it with a specific experiment. On the other hand, the two-dimensional markers (533) are placed, which allow the corners of the culture plate (104) and the features thereof to be defined.

In each particular embodiment, different types and combinations of codes can be used, placed in different positions on the culture plates (104).

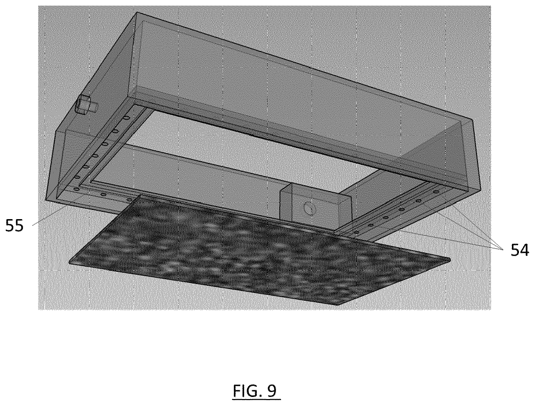

In addition, in accordance with an embodiment of the invention illustrated in Fig. 9, the growth monitoring module comprises diffuse lighting means (54). The diffuse lighting means (54) can be located at the edges of the filter holder (55).

In another embodiment of the invention illustrated in Fig. 1, the system comprises:
a control module (3) comprising:
aeration control means (31):
   comprising PID (proportional-integral-derivative) regulating means configured to maintain a given pressure in the air stirring module (8), the pressure being defined by a pressure transfer curve with respect to time, reading data from the pressure sensor (83) and acting on a speed of the flow control means (81), which can be a compressor;
   wherein the pressure regulating means comprise calculation means that allow problems in the air stirring system (8) to be detected, such as the detection of clogged ducts or a dirty filter;
air quality control means (32):
   comprising the integration of electronic sensors for detecting gases, such as CO₂ concentration, O₂ concentration, volatile organic compounds, etc.;
   These sensors are connected to the control module (3) by standardized interfaces (analogue ports, 12C port, SPI port);
organoid growth quantification means (33) configured to establish:
   growth of each of the organoids from the images obtained by the vision system, images that can be 2D images;
   different growth estimation algorithms, which can be adapted to different types of cultures by:
      - growth estimation by quantification of the maximum two-dimensional contour of different focal planes;
      - growth estimation by volumetric quantification on a 3D reconstruction of the organoid obtained from several 2D images captured in different focal planes;
real-time data capturing means (34) configured for:
   periodically sampling a reading of the value detected by the sensors connected to obtain captured data;
   saving the captured data on a long-term storage device, such as a flash memory;
      The sampling interval can be independently defined for each of the connected sensors;
   sending means for sending captured data to information systems (35):
      The captured data will be sent to the information systems through standard protocols, compatible with remote connection means. In case of using an ethernet or WiFi wireless network connection, data can be transmitted through standard TCP/IP protocols;
   remote connection means (36).

The control module (3) can be connected via wired or wireless connection. The following connection modes are supported, among others:
- Ethernet wired network
- WiFi wireless network (802.11)
- Bluetooth wireless connection
- Wired RS-232 serial connection
- Wired RS-485 serial connection
- Wired I2C serial connection
- Wired SPI serial connection

The control module (3) according to the invention may further comprise a system that provides real-time information on the aforementioned physical parameters: cell growth, CO₂ concentration, O₂ concentration, pH and temperature.

In an embodiment of the invention illustrated in Fig. 4, the culture module (2) and the growth monitoring module (50) are separated by a transparent base (15).

In another preferred embodiment, the number of sample wells (4) is an even number.

In another preferred embodiment, the number of sample wells (4) is 6, 8, 12, 24, 48 or 96. Preferably, the number of sample wells corresponds to the number of sample wells of a standard culture plate (104).

According to an embodiment of the invention, the system is adapted to be in an operating configuration and a stand-by configuration,
wherein in the operating configuration the stirring module (8) is coupled to the culture module (2) and the culture module (2) is coupled to the growth monitoring module (50);
and wherein in the stand-by configuration the culture module (2) is decoupled from the growth monitoring module (50) and from the stirring module (8), allowing the complete culture module (2) to be separated.

Another embodiment of the invention relates to a method for sterilizing an organoid culture system comprising:
providing a system like the one defined above in a stand-by configuration, such that the stirring module (8) and the growth monitoring module (50) are decoupled from the culture module (2);
removing the culture module (2);
and subjecting the culture module (2) to sterilization.

In an embodiment of the method of the invention, the sterilization to which the culture module (2) is subjected is autoclaving, treatment with hydrogen peroxide or treatment with ionizing radiation.

In another embodiment of the invention illustrated in Fig. 3, a container (1) comprises the culture module (2) and the growth monitoring module (50).

The real-time measurement of the aforementioned parameters, such as cell growth, CO₂ concentration, O₂ concentration, pH and temperature, are very advantageous for determining the correct growth of the organoid under suitable conditions.

With reference to Fig. 4, in a particular embodiment, the sample wells (4) are separated from the growth monitoring module (50) by a transparent base (15). This transparent base (15) allows the growth monitoring module (50) to be isolated in the event of a spill of culture medium and/or sample contained in the sample wells (4). In a preferred embodiment, the transparent base (15) is made of glass.

All the information collected from the control module (3) can be accessed at any time that the system is operating, but it can also be stored for subsequent analysis and/or quality control. The information can be stored locally, preferably on a memory card, and/or remotely, on the cloud using any standard network connection.

The system also preferably includes an alarm protocol that constantly alerts in the event of a system failure or an unexpected alteration of the physical parameters measured by the control module (3).

### EXAMPLES

### Example 1

The culture module (2) is coupled/decoupled from the growth monitoring module (50) as shown in Figs. 1 and 3.

### Example 2

The culture module (2) was sterilized as follows:
1. Autoclaved in a STERIVAP 669 - 1 ED (BMT) autoclave at 134 °C for 7 minutes.
2. 4 intensive drying phases of 3 minutes each.

### Example 3

The organoids were developed from two tumor cell lines previously obtained from primary RbloxP/loxP HRasV12 (T653) and cRb-/- HRasV12 (T731) astrocytes in SCID mice. These cells were cultured in DMEM (Dulbecco's Modified Eagle's Medium) supplemented with 10 % fetal bovine serum (FBS) at 37°C and 5% COD.

To establish the neurosphere culture derived from the primary tumor culture, the T653 and T731 cells were washed in phosphate-buffered saline (PBS) solution, trypsinized and recovered by centrifugation in PBS at 1000 rpm for 5 minutes. The cells were resuspended in the DMEM/F-12, GlutaMAX nutrient mix supplemented with 1X B-27 (50X) and 0.02 µg/ml EGF (Human Epidermal Growth Factor) and 0.02 µg/ml bFGF (basic fibroblast growth factor) growth factors. The cells were seeded in 60 mm plates and cultured at 37 °C and 5 % CO₂.

The cells were kept in a humidified incubator for 48 h, and after this time they were recovered by centrifugation at 1000 rpm for 5 min, resuspended in neural induction medium (DMEM/F-12 + GlutaMAX supplemented with 1 % N2 (100X), 1 % MEM-NEAA (MEM 100X non-essential amino acid solution) and 1 µg/ml heparin, seeded in 60 mm plates and kept in this culture medium for 48 hours at 37 °C and 5 % CO₂.

After this, the cells were cultured in Matrigel in 60 mm plates and in the presence of a differentiation culture medium. The composition of this medium was 50 % DMEM/F-12 + GlutaMAX and 50 % neurobasal medium (1X), supplemented with 0.5 % N2, 0.025 % Insulin, 0.5 % MEM-NEAA and 1 % penicillin-streptomycin, 0.035 % of 2- Mercaptoethanol (1: 1000 dilution) in DMEM/F-12 + GlutaMAX and 1 % B27 without vitamin A. The neurospheres were kept in Matrigel for 72 h before being transferred to the bioreactor.

The neurospheres were kept in the bioreactor in the presence of a differentiation medium supplemented with B27 with vitamin A (Lancaster MA *et al.,* 2014). The medium was changed every 72 h.

## Claims

1. A sterilizable organoid culture system comprising:
a culture module (2) comprising:
one or more sample wells (4);
an stirring module (8) comprising stirring means for each sample well (4);
**characterized in that**:
the stirring module (8) comprises:
an air compressor system comprising flow control means (81) configured to supply a pressurized air flow at a given pressure;
a nozzle (82) for each sample well (4), configured to channel the pressurized air flow to each sample well (4);
a pressure sensor (83) and a controller (84), the controller (84) being configured to act on the flow control means (81) by varying a supply power of the flow control means (81) based on a pressure reading provided by the pressure sensor (83).

2. A system according to claim 1, wherein the controller (84) is configured so that the pressure reading provided by the pressure sensor (83) corresponds to the pressure transfer/time curve selected for each experiment.

3. A system according to any of claims 1-2, wherein the stirring module (8) comprises:
a cover (5) comprising discharge means (51, 52) for discharging air from each sample well (4).

4. A system according to any of claims 1-3, wherein the stirring module (8) comprises:
an air filter (81) between each nozzle (82) and the air compressor system.

5. A system according to claim 4, wherein the air filter (81) is a HEPA filter.

6. A system according to any of claims 4-5, wherein the controller (84) is configured to detect a malfunction corresponding to a clogged nozzle (82), a dirty air filter (81), and both.

7. A system according to any of the preceding claims, wherein the sample wells (4) are arranged on support means (10).

8. A system according to any of the preceding claims, wherein the system comprises:
an environmental sensorization module (43) comprising one or more sensors (41);
wherein said one or more sensors (41) are configured to measure culture-related parameters selected from cell growth, CO₂ concentration, O₂ concentration, pH, temperature, humidity, and volatile organic compounds.

9. A system according to claim 8, wherein the one or more sensors (41) are configured to measure the culture-related parameters in real time.

10. A system according to any of claims 1-9, wherein the system comprises:
a growth monitoring module (50) comprising:
a vision system (511) based on individual images of each sample well (4).

11. A system according to claim 10, wherein the vision system (511) comprises image capturing means (51) selected from means comprising a fixed focus lens, adjustable focus lens, light field technology, and multi-camera technology.

12. A system according to any of claims 10-11, wherein the growth monitoring module (50) comprises a motorized system (52) for positioning the vision system (511) in each of the sample wells (4).

13. A system according to claim 12, wherein the motorized system (52) comprises a system selected from:
a Cartesian system with two motorized axes, X - Y, wherein:
the vision system (511) is installed on a sliding axis (X);
the sliding axis (X) is in turn installed on a perpendicular moving carriage (Y), driven by a mechanism independent of the X axis;
a Delta system with 3 motorized arms wherein:
the vision system (511) is installed on a support that is connected by three telescopic segments (521) to three carriages (522) configured to slide vertically on respective vertical axes (523), the vertical axes (523) being arranged on the vertices of an equilateral triangle seen on a horizontal plane;
the vision system is moved horizontally and vertically by a coordinated movement of the 3 carriages (522) along the vertical axes (523);
an articulated arm with at least two joints (52A, 52B) wherein the vision system (511) is located at a free end of the arm that has at least two rotating joints (52A, 52B), the coordinated movement of which allows the vision system to be positioned.

14. A system according to any of claims 12-13, wherein the motorized system (52) comprises calibrating means for calibrating the positioning of the vision system by means of visual guides (53) attached to a culture plate (104) selected from geometric shapes and visual codes.

15. A system according to claim 14, wherein the visual guides (53) are encoded and selected from two-dimensional codes and two-dimensional markers (fiducial markers).

16. A system according to any of claims 1-15, wherein the growth monitoring module comprises diffuse lighting means (54).

17. A system according to any of claims 10-16, wherein the system comprises:
a control module (3) comprising:
aeration control means (31):
comprising PID (proportional-integral-derivative) pressure regulating means configured to maintain a given pressure in the air stirring module (8), the pressure being defined by a pressure transfer curve with respect to time, reading data from the pressure sensor (83) and acting on a speed of the flow control means (81);
wherein the pressure regulating means comprise calculation means that allow problems in the air stirring module (8) to be detected;
air quality control means (32):
comprising the integration of electronic sensors for detecting gases, the sensors being connected to the control module (3) by standardized interfaces;
organoid growth quantification means (33) configured to establish:
growth of each of the organoids from images obtained by the vision system;
different growth estimation algorithms, which can be adapted to different types of cultures by:
- growth estimation by quantification of the maximum two-dimensional contour of different focal planes;
- growth estimation by volumetric quantification on a 3D reconstruction of the organoid obtained from several 2D images captured in different focal planes;
real-time data capturing means (34) configured for:
periodically sampling a reading of the value detected by the sensors connected to obtain captured data;
saving the captured data on a long-term storage device;
sending means for sending captured data to information systems (35) through standard protocols, compatible with remote connection means (36);
remote connection means (36) selected from wired and wireless connection, wherein the remote connection means (36) support at least one of the connection modes selected from ethernet wired network, WiFi wireless network (802.11), Bluetooth wireless connection, wired RS-232 serial connection, wired RS-485 serial connection, wired 12C serial connection, wired SPI serial connection, and combinations thereof.

18. The system according to any of claims 10-17, wherein the culture module (2) and the growth monitoring module (50) are separated by a transparent base (15).

19. The system according to any of the preceding claims, wherein the system is adapted to be in an operating configuration and a stand-by configuration,
wherein in the operating configuration the stirring module (8) is coupled to the culture module (2) and the culture module (2) is coupled to the growth monitoring module (50);
and wherein in the stand-by configuration the culture module (2) is decoupled from the growth monitoring module (50) and from the stirring module (8), allowing the complete culture module (2) to be separated.

20. A method for sterilizing an organoid culture system comprising:
providing a system like the one defined in claim 20 in a stand-by configuration, such that the stirring module (8) and the growth monitoring module (50) are decoupled from the culture module (2);
removing the culture module (2);
and subjecting the culture module (2) to sterilization.

21. The method according to claim 20, wherein the sterilization to which the culture module (2) is subjected is autoclaving, treatment with hydrogen peroxide or treatment with ionizing radiation.
